(19) Europäisches Patentamt

European Patent Office

Office européen des brevets

(11) **EP 1 079 203 B1**

(12) **EUROPÄISCHE PATENTSCHRIFT**

(45) Veröffentlichungstag und Bekanntmachung des Hinweises auf die Patenterteilung:
**04.12.2002 Patentblatt 2002/49**

(51) Int Cl.[7]: **G01B 7/06**, G01R 27/26, G01N 27/22, B05C 21/00

(21) Anmeldenummer: **00114600.0**

(22) Anmeldetag: **07.07.2000**

(54) **Kapazitive Überwachung des Leimauftrags auf ein Substrat mit der imaginären Permittivität**

Capacitive monitoring of the application of an adhesive on a substrate with imaginary permittivity

Surveillance capacitive d'application de colle sur un substrat avec la permittivité imaginaire

(84) Benannte Vertragsstaaten:
**AT BE CH CY DE DK ES FI FR GB GR IE IT LI LU MC NL PT SE**

(30) Priorität: **07.08.1999 DE 19937387**

(43) Veröffentlichungstag der Anmeldung:
**28.02.2001 Patentblatt 2001/09**

(73) Patentinhaber: **ITW Dynatec GmbH
40822 Mettmann (DE)**

(72) Erfinder: **Blank, Andreas
42553 Velbert (DE)**

(74) Vertreter: **Patentanwälte Ostriga & Sonnet
Stresemannstrasse 6-8
42275 Wuppertal (DE)**

(56) Entgegenhaltungen:
DE-A- 3 143 526    DE-A- 4 217 736
GB-A- 1 348 982    US-A- 3 766 469
US-A- 3 801 900    US-A- 4 288 741
US-A- 4 471 295    US-A- 5 001 435
US-A- 5 760 589

## Beschreibung

Die Erfindung betrifft eine Überwachungsvorrichtung eines Auftrags eines flüssigen bis pastenförmigen Mediums auf ein Substrat gemäß dem Oberbegriff des Anspruchs 1.

Eine derartige Vorrichtung ist in der DE 4217736 C2 beschrieben. Hier ist jede Elektrode als Sensor ausgebildet, der als Bestandteil eines Hochfrequenz-Schwingkreises eine Frequenzänderung erfährt, wenn sich eine Änderung der relativen Dielektrizität des Mediums ergibt, welches zwischen den beiden Sensoren angeordnet ist.

Der Sensor ist hier als kapazitiver Sensor, d.h. als Kapazität in den Hochfrequenz-Schwingkreis eingeschaltet. Je nach Art des zwischen den beiden Sonden befindlichen Mediums, d.h. abhängig davon, ob es sich um Luft, um eine Substrat ohne Leimnaht oder um ein Substrat mit einer unterschiedlichen dicken Leimnaht handelt, ändert sich die Kapazität dieser Anordnung. Die Kapazität der Anordnung ist jedoch stark abhängig von der relativen Dielektrizität der Materialien, welche für Luft, Papier und Leim stark unterschiedliche Werte annimmt.

Eine Änderung der Kapazität der Anordnung führt jedoch in dem bekannten Maße zu einer Änderung der Frequenz, woraus Rückschlüsse auf das Meßobjekt, d.h. das Substrat mit oder ohne Leimnaht gezogen werden können.

Die bekannte Vorrichtung zur Überwachung eines Auftrags eines flüssigen bis pastenförmigen Mediums auf ein Substrat erfüllt ihre Aufgaben sehr gut. Es kann in seltenen Fällen jedoch trotzdem zu Störungen kommen.

Der Erfindung liegt die Aufgabe zu Grunde, ausgehend von der bekannten Vorrichtung gemäß DE 4217736 C2 eine Vorrichtung gemäß dem Oberbegriff des Anspruchs 1 zu schaffen, die hinsichtlich Zuverlässigkeit und Meßgenauigkeit verbessert ist.

Die Erfindung löst diese Aufgabe mit den Merkmalen des Anspruchs 1, insbesondere denen des Kennzeichenteils, wonach die Vorrichtung zwischen den beiden Elektroden den komplexen Anteil der Permittivität des Substrats mit dem Medium mißt, und wonach die Meßelektonik diese Meßgröße zur Bestimmung des charakteristischen Signals verwendet.

Die Erfindung erkennt zunächst, daß die Permittivität, die auch Dielektrizität genannt wird, eine komplexe Größe ist, d.h. diese Größe weist einen Realteil und einen Imaginärteil auf. Weiterhin haben Versuche gezeigt, daß bei den hier interessierenden Materialien, insbesondere bei flüssigem bis pastenförmigem pastenförmigem Leim, wie er zur Herstellung einer Leimnaht auf einer Kartonage, auf Fliesmaterialien oder dgl. eingesetzt wird, die imaginären Anteile an der Permittivität größer, teilweise sogar um Größenordnung größer sind, als die realen Anteile an der Permittivität.

Daraus wurde erfindungsgemäß der Rückschluß gezogen, daß eine Messung des komplexen Anteils der Permittivität aufgrund dessen zahlenmäßig größeren Betrages eine einfachere und zuverlässigere Meßgröße hinsichtlich eines Rückschlusses auf das untersuchte Material darstellt.

Im folgenden wird kurz auf die formelmäßige Herleitung der Größen eingegangen. Weitere Einzelheiten können dem Scriptum von Herrn Professor Dr.-Ing. J. Prochotta, Frequenzzugang der komplexen Permittivität, FH-Düsseldorf, Labor Werkstoffkunde, 4. September 1998, Seiten 1 bis 14 entnommen werden.

Die einzelnen mikroskopischen Effekte, die sich bemerkbar machen, wenn sich ein dielektrisches Material in einem elektrischen Wechselfeld befindet, lassen sich am besten durch Einführung einer komplexen Permittivität gemäß folgender Formel deuten:

$$\underline{\varepsilon}_r = \varepsilon_r{}' - j\varepsilon{}''_r.$$

In obiger Formel bedeutet $\varepsilon_r{}'$ den Realteil, $\varepsilon_r{}''$ den Imaginärteil der Permittivität $\underline{\varepsilon}_r$.

Auf die einzelnen mikroskopischen Phänomene, die diese Größen beeinflussen, soll hier nicht weiter eingegangen werden. Grundsätzlich sei jedoch darauf hingewiesen, daß es sich um Effekte der Orientierungspolarisation, der ionischen Polarisation und der elektronischen Polarisation handelt. Die Permittivität sowie ihre beiden Anteile sind stark frequenzabhängig. Das $\varepsilon_r{}''$ beschreibt die dielektrischen Verluste und ist somit ein Maß für die vom Leim absorbierte Energie.

Diese dielektrischen Verluste verhalten sich wie ohmsche Wärmeverluste. Dies läßt sich auch mittels des sogenannten Verlustwinkels $\delta$ als Phasenverschiebung zwischen Strom und Spannung ausdrücken, so daß mit folgender Formel der dielektrische Verlustfaktor ausgedrückt ist:

$$\tan \delta = \frac{\varepsilon r''}{\varepsilon r'}$$

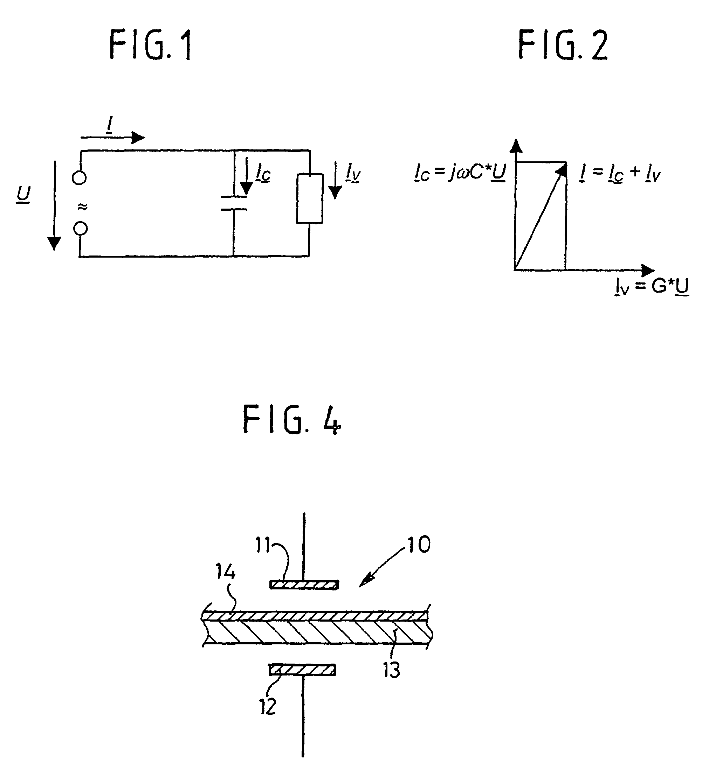

Verständlicher wird dies bei Betrachtung der Fig. 1 und 2. Fig. 1 zeigt schematisch ein Ersatzschaltbild eines realen, verlustbehafteten Kondensators. Beim Anlegen einer Wechselspannung $\underline{U}$ fließt durch den Konsensator der Strom I. Dieser umfaßt zwei Anteile, nämlich den Strom $\underline{I}_c$, der durch einen ideellen Kondensator fließen würde, sowie parallel dazu den Verluststrom $\underline{I}_v$, der als ohmscher Widerstand dargestellt die Anteile ausdrückt, die aufgrund der durch den komplexen Anteil der Permittivität erzeugten dielektrischen Verluste als Wärmeenergie im Kondensator verbleiben.

Fig. 2 zeigt als Diagramm die beiden Anteile von Verluststrom und Strom durch den ideellen Kondensator, die zusammenaddiert den Gesamtstrom I durch den reellen Kondensator ergeben.

Nach dem Ersatzschaltbild gilt nunmehr:

$$Y = G + j\omega C\,,$$

worin Y den Scheinleitwert, G den Verlustanteil und $j\omega C$ den Anteil durch den verlustfreien Kondensator darstellen.

Für den Fall, daß sich im Kondensator ein Meßobjekt befindet, gilt die Formel:

$$Y = j\omega * C_{Material}$$

Die Kapazität eines Plattenkondensators berechnet sich nach der Formel

$$C = \varepsilon \cdot \varepsilon r_d \frac{A}{}\,,$$

worin a die Fläche der Platten eines Plattenkondensators und d deren Abstand ist. $\varepsilon_r$ ist die Dielektrizität des Materials.

Aus den letzten beiden Formeln ergibt sich unmittelbar:

$$Y = j\omega * (\varepsilon_r' - j\varepsilon_r'') * \varepsilon_0 * \frac{A}{d}\,,$$

Wenn man im folgenden noch benutzt:

$$C_0 = \varepsilon_0 * \frac{A}{d}\,,$$

erhält man durch Vergleichen:

$$G + j\omega * C = j\omega * (\varepsilon_r' - j\varepsilon_r'') * C_0$$

$$G + j\omega * C = \omega * \varepsilon_r'' * C_0 + j\omega * \varepsilon_r' * C_0\,.$$

daraus erhält man nunmehr unmittelbar:

$$C = \varepsilon_r' * C_0.$$

Dies zeigt jedoch, daß in die Kapazität lediglich der Realteil der Permittivität eingeht. Die im Stand der Technik bisher übliche Kapazitätsmessung liefert daher nicht den komplexen Anteil der Permittivität.

Weiterhin wird nunmehr auf die Tabellen 1 und 2 verwiesen.

Tabelle 1

| $C_r$/pF | 1,4 |
|---|---|
| $C_s$/pF | 0,2 |

| f/MHz | 0,075 | 0,1 | 0,15 | 0,2 | 0,3 | 0,5 | 1 |
|---|---|---|---|---|---|---|---|
| C/pF | 100,28 | 99,80 | 99,17 | 98,74 | 98,16 | 97,44 | 96,41 |
| $C_K$/pF | 98,48 | 98,0 | 97,37 | 96,94 | 96,46 | 95,64 | 94,61 |
| G/µS | 1,38 | 1,74 | 2,43 | 3,10 | 4,42 | 7,03 | 13,18 |
| $C_L$/pF | 18,93 | 18,93 | 18,93 | 18,92, | 18,92 | 18,92 | 18,92 |
| $C_{LK}$/pF | 17,13 | 17,13 | 17,13 | 17,12 | 17,12 | 17,12 | 17,12 |
| $\varepsilon_r'$ | 5,68 | 5,72 | 5,68 | 5,66 | 5,63 | 5,58 | 5,52 |
| $\varepsilon_r''$ | 0,17 | 0,16 | 0,15 | 0,14 | 0,14 | 0,13 | 0,12 |
| tan δ | 0,03 | 0,028 | 0,026 | 0,025 | 0,025 | 0,023 | 0,022 |

Tabelle 2

| $C_r$/pF | 1,4 |
|---|---|
| $C_s$/pF | 0,2 |

| f/MHz | 0,075 | 0,1 | 0,15 | 0,2 | 0,3 | 0,5 | 1 |
|---|---|---|---|---|---|---|---|
| C/pF | 1187 | 965 | 879 | 653 | 548 | 459 | 381 |
| CK/pF | 1185,4 | 963,4 | 877,4 | 651,4 | 546,4 | 457,4 | 379,4 |
| G/µS | 4074 | 4163 | 4293 | 4374 | 4447 | 4620 | 4935 |
| CL/pF | 16,55 | 16,55 | 16,55 | 16,54 | 16,54 | 16,54 | 16,54 |
| CLK/pF | 14,95 | 14,95 | 14,95 | 14,94 | 14,94 | 14,94 | 14,94 |
| εr' | 79,3 | 64,44 | 58,7 | 43,6 | 36,6 | 30,6 | 25,4 |
| εr'' | 578,3 | 443,2 | 304,7 | 233,0 | 157,9 | 98,4 | 52,6 |
| tan δ | 7,29 | 6,88 | 5,19 | 5,34 | 4,31 | 3,22 | 2,07 |

Tabelle 1 zeigt eine Vielzahl von gewonnenen Meßwerten, bei denen Papier zwischen die beiden Platten eines Plattenkondensators gebracht wurde. Tabelle 2 zeigt die entsprechenden Meßgrößen, bei denen das zwischen den beiden Platten des Plattenkondensators eingebrachte Dielektrikum aus zwei Papierschichten mit dazwischen angeordneten Leimschichten bestand.

In den Tabellen bedeuten im Einzelnen: $C_r$ den Kapazitätsanteil der Kapazität der gemessenen Probe, der das Randfeld des Plattenkondensators berücksichtigt, $C_s$ den Kapazitätsanteil, der das Streufeld gegen die Erde berücksichtigt, f die angelegte Frequenz, C die gemessene Kapazität, $C_K$ die korrigierte gemessene Kapazität, G den Leitwert des Dielelektrikums, $C_L$ die Kapazität der Meßanordnung ohne Probe, $C_{LK}$ die korrigierte Kapazität der

Meßanordnung ohne Probe, $\varepsilon_r'$ den Realteil der Permittivität, $\varepsilon r''$ den komplexen Anteil der Permittivität, und tan $\delta$ den Verlustwinkel.

Weitere Einzelheiten zu den Messungen kann man ebenfalls dem oben angegebenen Scriptum von Prof. Dr. -Ing. J. Prochetta entnehmen.

Den Tabellen entnimmt man ohne weiteres, daß im Falle von Leim als Dielektrikum die absoluten Werte für den komplexen Anteil $\varepsilon_r''$ der Permittivität um Größenordnungen über dem realen Anteil $\varepsilon_r'$ der Permittivität liegen. Damit wird deutlich, daß eine Messung dieses komplexen (oder imaginären) Anteils der Permittivität eine deutlich bessere Aussage über das zwischen den beiden Elektroden befindliche Dielektrikum zuläßt. Hierin liegt der Kern der Erfindung.

Weiterhin wird deutlich, daß das Verhältnis $\varepsilon_r''$ (Leim) zu $\varepsilon_r''$ (Papier) um mehr als zwei Größenanordnungen über dem Verhältnis $\varepsilon_r'$ (Leim) zu $\varepsilon_r'$ (Papier) liegt.

Aus der GB-A-1348982 ist eine Vorrichtung bekannt, die den Leimauftrag mittels einer zwei oder drei Elektroden umfassenden Anordnung überwacht. Um festzustellen, ob die Klebenaht vorhanden ist oder nicht, wird ein gepulstes Signal an einer Elektrode angelegt, und an einer zweiten Elektrode das induzierte Signal gemessen. Die Messung des komplexen, also imaginären Anteils der Permittivität von Substrat mit Medium und der Verwendung dieser Messgröße zur Bestimmung des charakteristischen Signals ist dieser Schrift nicht zu entnehmen.

Aus der US-A-3766469 ist eine Vorrichtung bekannt, mittels der die Kapazität und der Verlustwinkel von Komponenten eines Stromleitungsnetzes gemessen werden können.

Gemäß einer vorteilhaften Ausgestaltung der Erfindung erfolgt zur Messung des komplexen Anteil der Permittivität eine Strommessung bzw. eine Stromabnahmemessung durch das Substrat um das Medium hindurch. Dies trägt der Tatsache Rechnung, dass in einer Strommessung der komplexe Anteil der Permittivität des Dielektrikums besonders einfach zu bestimmen ist. Dies ergibt sich auch aus den obigen Formeln, denn durch analoges Vergleichen erhält man die Formel:

$$G = \omega * \varepsilon_r'' * C_0.$$

Hier wird ersichtlich, daß der Verlustanteil G direkt proportional zum komplexen Anteil der Permittivität ist. Die Messung dieses Verluststrom liefert also unmittelbar das gewünschte Ergebnis.

Gemäß einer weiteren vorteilhaften Ausgestaltung der Erfindung erfolgt die Strommessung bzw. Stromabnahmemessung durch einen stromgesteuerten Spannungsverstärker, insbesondere durch einen I-/U-Wandler-Baustein. Dies ermöglicht auf einfache und vorteilhafte Weise die Messung von vorliegend sehr geringen Strömen.

Gemäß einer weiteren vorteilhaften Ausgestaltung ist der I-/U-Wandler-Baustein mit einem Addierer verbunden, der seinerseits mit dem Ausgang eines ersten Operationsverstärkers verbunden ist. Dies ermöglicht eine vorteilhafte Weiterverarbeitung des ermittelten Signals am Ausgang I-/U-Wandlers auf einfache Weise.

Gemäß einer weiteren Ausgestaltung der Erfindung ist zwischen dem Eingang des ersten Operationsverstärkers und einer Wechselspannungsquelle ein erster Phasenschieber angeordnet. Diesem kommt die Aufgabe zu, die am Ausgang des I-/U-Wandlers vorliegende Phasenverschiebung zwischen Strom und Spannung zu kompensieren.

Gemäß einer weiteren vorteilhaften Ausgestaltung der Erfindung ist der Phasenschieber phaseninvertiert. Damit läßt sich die Vorrichtung derart einstellen, daß ein Abgleich bei leeren Sensor, d.h. ohne Substrat und ohne Medium ein Messergebnis am Ausgang des Addierers von 0 liefert. Auf diese Weise lassen sich die geringen, gemessenen Spannungen in vorteilhafter Weise weiter verarbeiten, wenn sich ein Dielektrikum zwischen den Elektroden befindet.

Gemäß einer weiteren vorteilhaften Ausgestaltung der Erfindung umfaßt der I-/U-Wandler eine Schaltung mit einem dritten Operationsverstärker. Dies ermöglicht eine preiswerte und einfache Herstellung eines I-/U-Wandlers.

Gemäß einer weiteren vorteilhaften Ausgestaltung der Erfindung ist ein erster Eingang des dritten Operationsverstärkers, insbesondere der invertierende Eingang, unmittelbar mit einer der Elektroden des Sensors verbunden. Dies ermöglicht eine Signalabnahme, ohne daß störende Einflüsse zu befürchten sind.

Gemäß einer weiteren vorteilhaften Ausgestaltung sind die beiden Elektroden auf unterschiedlichen Seiten des Substrats angeordnet. Damit wird eine Anordnung möglich, die nach Art eines Plattenkondensators zwei plattenförmige Elektroden umfaßt, zwischen denen das Substrat mit oder ohne Medium hindurch bewegt wird. Außerdem ermöglicht dieser Aufbau eine Messung durch das Substrat und das Medium hindurch, quer zur Substratebene. Dadurch wird die Messung besonders einfach.

Eine andere alternative vorteilhafte Ausgestaltung ist die Anordnung der beiden Elektroden auf einer Seite des Substrats. Hier findet die Messung des komplexen Anteils der Permittivität ebenfalls zwischen den beiden Elektroden statt, nunmehr zumindest teilweise in einer ebene parallel zur Ebene der Substratoberfläche.

Weitere Vorteile der Erfindung ergeben sich aus den nicht zitierten Unteransprüchen sowie anhand der Beschreibung des in den Figuren dargestellten Ausführungsbeispiels der Erfindung. Es zeigen:

Fig. 1 das Ersatzschaltbild eines realen, verlustbehafteten Kondensators,

Fig. 2 das Stromdiagramm zur Veranschaulichung der beiden Stromanteile gemäß Ersatzschaltbild von Fig. 1,

Fig. 3 das Blockschaltbild einer Meßelektronik für die erfindungsgemäße Vorrichtung,

Fig. 4 schematisch den Bereich eines Sensors mit zwei plattenförmig ausgebildeten Elektroden sowie einer dazwischen angeordneten Materialbahn und

Fig. 5 ein detaillierteres Schaltbild der Meßelektronik gemäß Fig. 3.

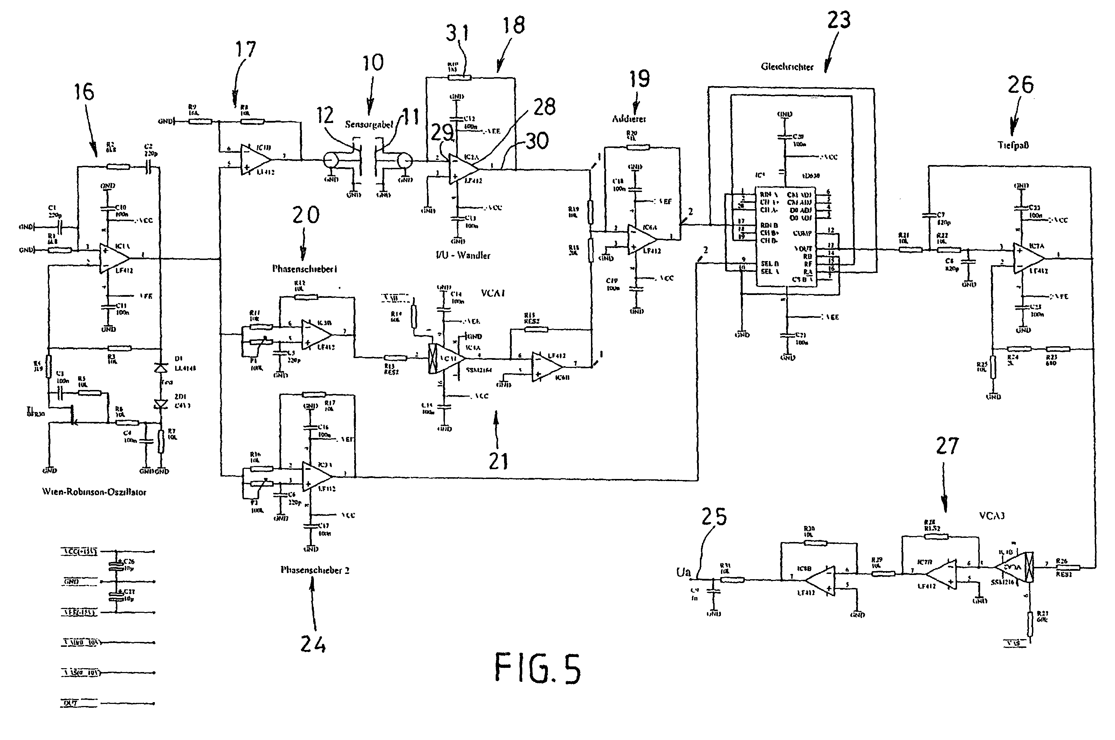

Die in Ihrer Gesamtheit nicht dargestellte Vorrichtung zur Überwachung eines Auftrags eines flüssigen bis pastenförmigen Mediums auf ein Substrat umfaßt erfindungsgemäß einen Sensor 10, der in Fig. 4 lediglich schematisch dargestellt ist. Beim Ausführungsbeispiel gemäß Fig. 4 handelt es sich bei dem Sensor 10 im Prinzip um einen Plattenkondensator, dessen beide Elektroden 11 und 12 im wesentlichen plattenförmig ausgebildet sind. Zwischen den beiden Elektroden 11, 12 befindet sich gemäß Fig. 4 eine Materialbahn 13, die auf ihrer der oberen Elektrode 11 zugewandten Seite einen Leimauftrag 14 umfaßt.

Hinsichtlich des allgemeinen Aufbaus, der Betriebsweise und der damit verbundenen Probleme bei Leimauftrags-Überwachungs Vorrichtungen wird pauschal auf die DE 4217736C2 und die DE 3934852C2 verwiesen.

Die erfindungsgemäße Vorrichtung unterscheidet sich von den in den beiden Druckschriften beschriebenen im wesentlichen durch die Art des verwendeten Sensors und die Meßweise bzw. die Meßelektronik.

Überwacht werden soll mittels der erfindungsgemäßen Vorrichtung ebenfalls der Auftrag eines Mediums, insbesondere eines Leims, um ordnungsgemäße Leimnähte auf Kartonagen, auf aus Fliesmaterialien und dünnen Kunststoffolien aufgebauten Windeln oder dgl. in einer Fertigungsstraße festzustellen bzw. bei nicht ordnungsgemäßen Leimnähten entsprechend einen Alarm o.ä. zu verursachen.

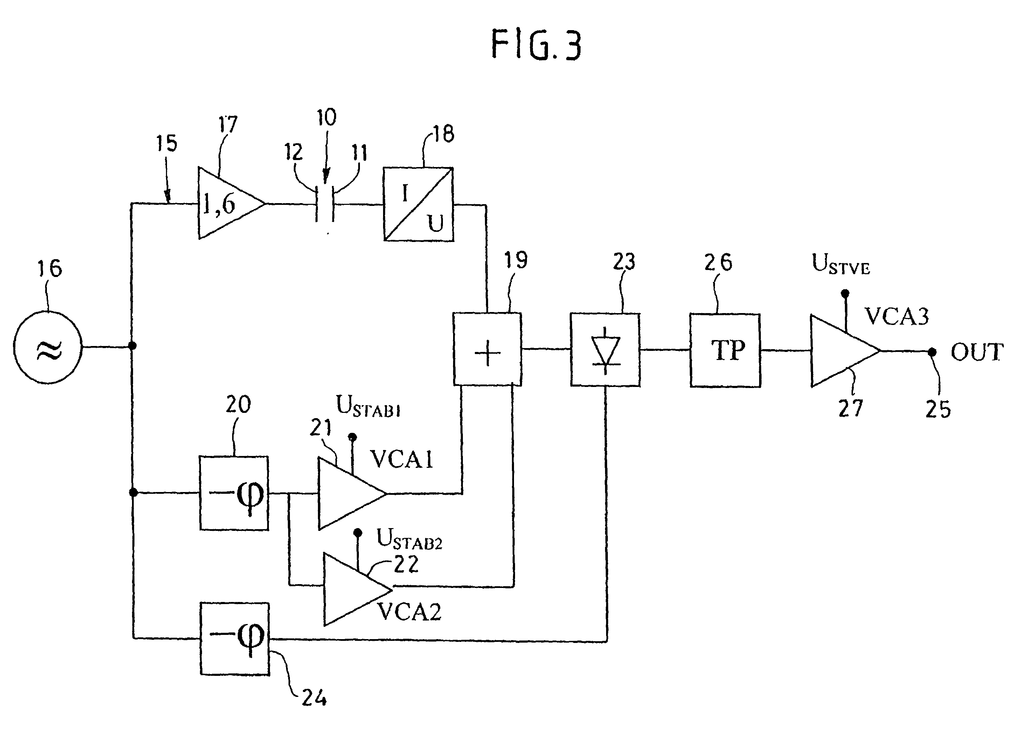

Gemäß Fig. 3 befindet sich der Sensor 10, der auch als Sensorgabel bezeichnet wird, in einem pauschal mit 15 bezeichneten Meßkreis. Dieser umfaßt zunächst eine Wechselspannungsquelle 16, die beim Ausführungsbeispiel gemäß Fig. 3 und 5 als Wien-Robinson-Oszillator ausgebildet ist.

Dieser ist zunächst mit einem Verstärker 17 verbunden, der beispielsweise eine 1,6fache Verstärkung erreicht, bevor das Wechselspannungssignal an der ersten, gemäß Fig. 4 unteren Elektrode 12, anliegt. Die gegenüberliegende Elektrode 11, also gemäß Ausführungsbeispiel von Fig. 4 die obere Eiektrode 11, ist unmittelbar mit einem I-/U-Wandler-Baustein 18 verbunden (Fig. 3). Dieser mißt den Verluststrom durch den Sensor 10.

Der Ausgang des I-/U-Wandler-Baustein 18 ist mit einem Addierer 19 verbunden.

Von der Wechselspannungsquelle 16 aus führt ein zweiter Leitungszweig zu einem ersten, invertierten Phasenschieber 20. Dessen Ausgang ist mit dem Eingang eines ersten Operationsverstärkers 21 verbunden. Der Ausgang des ersten Operationsverstärkers 21 führt in den Eingang des Addierers 19.

Sinn und Zweck des gerade beschriebenen Zweiges ist es, mittels des ersten Phasenschiebers 20 die sinusförmige Wechselspannung an den phasenversetzt verlaufenden Ausgang des I-/U-Wandler-Bausteins 18 anzupassen.

Durch Addieren des Ausgangswertes des ersten Operationsverstärkers 21 und des Ausgangs des I-/U-Wandler-Bausteins 18 erhält man prinzipiell bereits die gewünschte Meßgröße, nämlich den Verluststrom durch den Sensor 10 hindurch.

Um die Messung jedoch noch etwas eleganter auszuführen, sind noch folgende weitere Elemente vorgesehen: Der Ausgang des ersten Phasenschiebers 20 ist mit dem Eingang eines zweiten Operationsverstärkers 22 verbunden. Dessen Ausgang ist wiederum mit dem Addierer 19 verbunden. Die Bedeutung dieses Zweiges wird später deutlich.

Weiterhin ist der Ausgang des Addierer 19 mit einem Gleichrichter-Bauelement 23 verbunden. Zwischen dem Gleichrichter-Bauelement 23 und der Wechselspannungsquelle 16 ist über einem separaten Zweig ein zweiter, invertierter Phasenschieber 24 angeordnet.

Nunmehr wird klar, inwieweit die letzten beiden beschriebenen Zweige zusammenspielen: Mit Hilfe des zweiten Phasenschiebers 24 läßt sich nämlich die Wechselspannung hinter dem Addierer 19, also am Ausgang des Addierers 19, derart anpassen, daß man am Ausgang des Gleichrichters 23 immer eine positive Gleichspannung erhält. Dieses hat bei der Weiterverarbeitung des Signals selbstverständlich Vorteile.

Weiterhin ist zwischen dem Gleichrichter-Bauelement 23 und dem Ausgang 25 der Meßelektronik 25 ein Tiefpaß 26 vorgesehen. Auch dieser bietet prinzipiell bekannte, meßtechnische Vorteile.

Schließlich ist zur Verstärkung des Ausgangssignals noch ein vierter Operationsverstärker 27 vorgesehen.

Eine detailgetreuere Darstellung der Meßelektronik gemäß Fig. 3 befindet sich in Fig. 5. Gleiche Teile oder Baugruppen wurden mit den gleichen Bezugszeichen bezeichnet. Hervorzuheben ist, daß der zweite Operationsverstärker 22, der in Fig. 3 dargestellt ist, hier fehlt. Dieser ist prinzipiell auch nicht notwendig.

Hervorzuheben ist in Fig. 5 insbesondere die detaillierte Bauweise des I-/U-Wandler-Bausteins 18. Dieser umfaßt im wesentlichen einen Operationsverstärker 28.

Auf diese Weise kann ein herkömmlicher, kommerziell erhältlicher Baustein, insbesondere der LF412, verwendet werden, der auch in den übrigen Bauelementen, wie Phasenschieber 20, 24, erster Operationsverstärker 21, vierter Operationsverstärker 27, Addierer 19, Tiefpaß 26 und auch in der Wechselspannungsquelle 16 Verwendung findet.

Die Angaben der Kapazitäten und Widerstände in dem Schaltbild sind weitgehend korrekt. Es versteht sich jedoch von selbst, daß die einzelnen, genauen Angaben lediglich beispielhaft zu nehmen sind.

Hervorzuheben ist, daß zwischen dem invertierten Eingang 29 des dritten Operationsverstärker 28 und dem Ausgang 30 des dritten Operationsverstärker 28 ein Widerstand 31 von einem Megaohm angeordnet ist.

Weiterhin ist hervorzuheben, daß die Elektroden 11, 12 des Sensors 10 gemäß Ausführungsbeispiel zwar nach Art eines Plattenkondensator ausgebildet sind. Der Erfindungsgedanke ist jedoch nicht auf Elektroden derartiger Bauformen beschränkt. Prinzipiell ist zunächst einmal jede geometrische -Bauform der Elektroden möglich und geeignet, zwischen sich den komplexen Anteil der Permittivität zu messen.

An den Phasenschiebern kann jeweils eine Phasenverschiebung von 0 bis 170° eingestellt werden. Dies dient im wesentlichen nur dem Abgleich der Ausgangswerte. Den gleichen Zweck dienen auch die Steuerspannungen USTAB1 und USTAB2, die an dem ersten und an dem zweiten Operationsverstärker 21 und 22 anliegen (Fig. 3). Der Abgleich erfolgt dann, wenn sich kein Dielektrikum zwischen den Elektroden befindet. Die Steuerspannungen sind dabei zwischen 0 und 10 Volt stufenlos einstellbar.

Die angelegte Meßfrequenz beträgt beispielsweise 100 kHz. Es sind aber jedoch Frequenzen im Bereich einiger kHz bis zu einigen 10 oder 100 MHz denkbar. Die genaue Frequenz richtet sich insbesondere nach dem zu messenden Produkt, und dabei insbesondere nach dem aufzutragenden flüssigen bis pastenförmigen Medium. Je nach Leimart kann der komplexe Anteil der Permittivität bzw. daß Verhältnis von komplexer zu realer Permittivität unterschiedliche Werte annehmen. Eine einmal eingestellte Frequenz bleibt dann jedoch während des Überwachungsbetriebes in der Regel konstant.

Der in den Fig. 3 und 5 dargestellte Verstärker 17 ist lediglich optional. Er hat sich jedoch als vorteilhaft erwiesen.

**Patentansprüche**

1. Überwachungsvorrichtung eines Auftrags eines flüssigen bis pastenförmigen Mediums (14) auf ein Substrat (13), mit wenigstens einem Sensor (10), der zwei Elektroden (11,12) umfaßt, die unter einem bestimmten, geringen Abstand zu der Substratoberfläche angeordnet sind, und die das Substrat (13) und das Medium (14) mit einer Wechselspannung beaufschlagen, wobei das Substrat (13) eine Bewegung relativ zu dem Sensor (10) durchläuft, und mit einer Meßelektronik, die mit dem Sensor (10) verbunden ist, und die ein für das Substrat (13) mit dem Medium (14) charakteristisches Signal ausgibt, **dadurch gekennzeichnet, daß** die Vorrichtung zwischen den beiden Elektroden (11,12) den imaginären Anteil der Permittivität des Substrats (13) mit dem Medium (14) mißt, und daß die Meßelektronik (15) diese Meßgröße zur Bestimmung des charakteristischen Signals verwendet.

2. Vorrichtung nach Anspruch 1, **dadurch gekennzeichnet, daß** zur Messung des komplexen Anteils der Permittivität eine Strommessung bzw. eine Stromabnahmemessung durch das Substrat (13) und das Medium (14) hindurch erfolgt.

3. Vorrichtung nach Anspruch 2, **dadurch gekennzeichnet, daß** die Strommessung bzw. Stromabnahmemessung durch einen stromgesteuerten Spannungsverstärker (18), insbesondere durch einen I-/U-Wandler-Baustein, erfolgt.

4. Vorrichtung nach Anspruch 3, **dadurch gekennzeichnet, daß** der I-/U-Wandler (18) mit einem Addierer (19) verbunden ist, der seinerseits mit dem Ausgang eines ersten Operationsverstärkers (21) verbunden ist.

5. Vorrichtung nach einem der vorangegangenen Ansprüche, **dadurch gekennzeichnet, daß** zur Erzeugung der Wechselspannung eine Wechselspannungsquelle (16) vorgesehen ist.

6. Vorrichtung nach einem der Ansprüche 4 bis 5, **dadurch gekennzeichnet, daß** zwischen dem Eingang des ersten Operationsverstärkers (21) und der Wechselspannungsquelle (16) ein erster Phasenschieber (20) angeordnet ist.

7. Vorrichtung nach Anspruch 6, soweit dieser auf Anspruch 4 rückbezogen ist, **dadurch gekennzeichnet, daß** zwischen dem ersten Phasenschieber (20) und dem Addierer (19) ein zweiter Operationsverstärker (22) angeordnet ist.

8. Vorrichtung nach einem der Ansprüche 4 bis 7, **dadurch gekennzeichnet, daß** zwischen dem Addierer (19) und dem Ausgang der Meßelektronik (25) ein Gleichrichter-Baustein (23) angeordnet ist.

9. Vorrichtung nach Anspruch 8, **dadurch gekennzeichnet, daß** zwischen dem Gleichrichter-Baustein (23) und der Wechselspannungsquelle (16) ein zweiter Phasenschieber (24) angeordnet ist.

10. Vorrichtung nach einem der Ansprüche 6 bis 9, **dadurch gekennzeichnet, daß** der oder die Phasenschieber phaseninvertiert sind.

11. Vorrichtung nach einem der Ansprüche 9 bis 10, **dadurch gekennzeichnet, daß** zwischen dem Gleichrichter-Baustein (23) und dem Ausgang der Meßelektronik (25) ein Tiefpaß (26) angeordnet ist.

12. Vorrichtung nach einem der Ansprüche 5 bis 11, **dadurch gekennzeichnet, daß** zwischen dem Sensor (10) und der Wechselspannungsquelle (16) ein Verstärkerbaustein (17) angeordnet ist.

13. Vorrichtung nach einem der Ansprüche 3 bis 12, **dadurch gekennzeichnet, daß** der I-/U-Wandler (18) eine Schaltung mit einem dritten Operationsverstärker (28) umfaßt.

14. Vorrichtung nach Anspruch 13, **dadurch gekennzeichnet, daß** ein erster Eingang (29) des dritten Operationsverstärkers (28), insbesondere der invertierende Eingang, unmittelbar mit einer der Elektroden (11) des Sensors (10) verbunden ist.

15. Vorrichtung nach Anspruch 13 oder 14, **dadurch gekennzeichnet, daß** zwischen dem ersten Eingang (29) des dritten Operationsverstärkers (28) und dessen Ausgang (30) ein hoher Widerstand (31), insbesondere in der Größenordnung 1 Megaohm, angeordnet ist.

16. Vorrichtung nach einem der Ansprüche 5 bis 15, **dadurch gekennzeichnet, daß** die Wechselspannungsquelle (16) ein Wien-Robinson-Oszillator ist.

17. Vorrichtung nach einem der vorangegangenen Ansprüche, **dadurch gekennzeichnet, daß** die beiden Elektroden (11, 12) auf unterschiedlichen Seiten des Substrates (13) angeordnet sind.

18. Vorrichtung nach einem der Ansprüche 1 bis 17, **dadurch gekennzeichnet, daß** die beiden Elektroden (11,12) auf einer Seite des Substrates (13) angeordnet sind.

## Claims

1. Monitoring device for application of a liquid to paste-like medium (14) to a substrate (13), with at least one sensor (10) comprising two electrodes (11, 12) which are arranged at a specific short distance from the substrate surface and which apply an alternating voltage to the substrate (13) and the medium (14), wherein the substrate (13) performs a movement relative to the sensor (10), and with a measurement electronic unit connected with the sensor (10) which emits a signal characteristic for the substrate (13) with the medium (14), **characterised in that** between the two electrodes (11, 12) the device measures the imaginary part of the permittivity of the substrate (13) with the medium (14) and that the measurement electronic unit (15) uses this measurement parameter to determine the characteristic signal.

2. Device according to claim 1, **characterised in that** to measure the complex part of the permittivity the current or current reduction through the substrate (13) and the medium (14) is measured.

3. Device according to claim 2, **characterised in that** the current or current reduction is measured by a current-controlled voltage amplifier (18), in particular an I/U converter module.

4. Device according to claim 3, **characterised in that** the I/U converter (18) is connected to an adder (19) which in turn is connected with the output of a first operation amplifier (21).

**5.** Device according to any of the preceding claims, **characterised in that** to generate the alternating voltage, an alternating voltage source (16) is provided.

**6.** Device according to any of claims 4 to 5, **characterised in that** between the input of the first operation amplifier (21) and the alternating voltage source (16) is arranged a first phase shifter (20).

**7.** Device according to claim 6, where this relates back to claim 4, **characterised in that** between the first phase shifter (20) and the adder (19) is arranged a second operation amplifier (22).

**8.** Device according to any of claims 4 to 7, **characterised in that** between the adder (19) and the output from the measurement electronic unit (25) is arranged a rectifier module (23).

**9.** Device according to claim 8, **characterised in that** between the rectifier module (23) and the alternating voltage source (16) is arranged a second phase shifter (24).

**10.** Device according to any of claims 6 to 9, **characterised in that** the phase shifter or shifters are phase-inverted.

**11.** Device according to any of claims 9 to 10, **characterised in that** between the rectifier module (23) and the output from the measurement electronic unit (25) is arranged a low pass filter (26).

**12.** Device according to any of claims 5 to 11, **characterised in that** between the sensor (10) and the alternating voltage source (16) is arranged an amplifier module (17).

**13.** Device according to any of claims 3 to 12, **characterised in that** the I/U converter (18) comprises a circuit with a third operation amplifier (28).

**14.** Device according to claim 13, **characterised in that** a first input (29) of the third operation amplifier (28), in particular the inverting input, is directly connected with one of the electrodes (11) of the sensor (10).

**15.** Device according to claim 13 or 14, **characterised in that** between the first input (29) of the third operation amplifier (28) and its output (30) is arranged a high resistance (31), in particular of the order of 1 Megaohm.

**16.** Device according to any of claims 5 to 15, **characterised in that** the alternating voltage source (16) is a Wien-Robinson oscillator.

**17.** Device according to any of the preceding claims, **characterised in that** the two electrodes (11, 12) are arranged on different sides of the substrate (13).

**18.** Device according to any of claims 1 to 17, **characterised in that** the two electrodes (11, 12) are arranged on one side of the substrate (13).


**Revendications**

**1.** Dispositif de surveillance d'une application d'un milieu de transport (14), de caractéristique fluide à pâteuse, sur un substrat (13), avec au moins un capteur (10), comprenant deux électrodes (11, 12) disposées sous un faible espacement déterminé par rapport à la surface du substrat et sollicitant par une tension alternative le substrat (13) et le milieu (14), le substrat (13) effectuant un déplacement par rapport au capteur (10), et avec une électronique de mesure, reliée au capteur (10) et qui envoie un signal caractéristique du substrat (13) avec le milieu (14), **caractérisé en ce que** le dispositif mesure entre les deux électrodes (11, 12) la fraction imaginaire de la permittivité du substrat (13) avec le milieu (14), et **en ce que** l'électronique de mesure (15) utilise cette grandeur de mesure pour déterminer le signal caractéristique.

**2.** Dispositif selon la revendication 1, **caractérisé en ce que**, pour effectuer la mesure de la fraction complexe de la permittivité, on effectue une mesure d'intensité ou une mesure de diminution d'intensité à travers le substrat (13) et le milieu (14).

**3.** Dispositif selon la revendication 2, **caractérisé en ce que** la mesure d'intensité ou la mesure de diminution d'intensité est effectuée à l'aide d'un amplificateur de tension (18) à commande en intensité, en particulier en utilisant un composant convertisseur I/U.

4. Dispositif selon la revendication 3, **caractérisé en ce que** le convertisseur I/U (18) est relié à un additionneur (19), relié de son côté à la sortie d'un premier amplificateur opérationnel (21).

5. Dispositif selon l'une des revendications précédentes, **caractérisé en ce qu'**une source de tension alternative (16) est prévue pour produire la tension alternative.

6. Dispositif selon l'une des revendications 4 à 5, **caractérisé en ce qu'**entre l'entrée du premier amplificateur opérationnel (21) et la source de tension alternative (16) est disposé un premier déphaseur (20).

7. Dispositif selon la revendication 6, dans la mesure où celle-ci se réfère à la revendication 4, **caractérisé en ce qu'**entre le premier déphaseur (20) et l'additionneur (19) est disposé un deuxième amplificateur opérationnel (22).

8. Dispositif selon l'une des revendications 4 à 7, **caractérisé en ce qu'**entre l'additionneur (19) et la sortie de l'électronique de mesure (25) est disposé un composant redresseur (23).

9. Dispositif selon la revendication 8, **caractérisé en ce qu'**entre le composant redresseur (23) et la source de tension alternative (16) est disposé un deuxième déphaseur (24).

10. Dispositif selon l'une des revendications 6 à 9, **caractérisé en ce que** le ou les déphaseurs est/sont inversé(s) en phase.

11. Dispositif selon l'une des revendications 9 à 10, **caractérisé en ce qu'**entre le composant redresseur (23) et la sortie de l'électronique de mesure (25) est disposé un filtre passe-bas (26).

12. Dispositif selon l'une des revendications 5 à 11, **caractérisé en ce qu'**entre le capteur (10) et la source de tension alternative (16) est disposé un composant amplificateur (17).

13. Dispositif selon l'une des revendications 3 à 12, **caractérisé en ce que** le convertisseur I/U (18) comprend un circuit ayant un troisième amplificateur opérationnel (28).

14. Dispositif selon la revendication 13, **caractérisé en ce qu'**une première entrée (29) du troisième amplificateur opérationnel (28), en particulier l'entrée inversive, est directement reliée à l'une des électrodes (11) du capteur (10).

15. Dispositif selon la revendication 13 ou 14, **caractérisé en ce qu'**entre la première entrée (29) du troisième amplificateur opérationnel (28) et sa sortie (30) est disposée une résistance (31) de valeur élevée, en particulier de l'ordre de grandeur de 1 Mégaohm.

16. Dispositif selon l'une des revendications 5 à 15, **caractérisé en ce que** la source de tension alternative (16) est un oscillateur Wien-Robinson.

17. Dispositif selon l'une des revendications précédentes, **caractérisé en ce que** les deux électrodes (11, 12) sont disposées sur des côtés différents du substrat (13).

18. Dispositif selon l'une des revendications 1 à 17, **caractérisé en ce que** les deux électrodes (11, 12) sont disposées sur un côté du substrat (13).

# FIG.1

# FIG.2

$$\underline{I}_C = j\omega C^{\star}\underline{U} \qquad \underline{I} = \underline{I}_C + \underline{I}_v$$

$$\underline{I}_v = G^{\star}\underline{U}$$

# FIG. 4

# FIG.3

EP 1 079 203 B1

FIG.5

EP 1 079 203 B1